# EUROPEAN PATENT SPECIFICATION

(11) **EP 1 416 885 B1**
(45) Date of publication and mention of the grant of the patent: **10.01.2007**
(21) Application number: 02763362.7
(22) Date of filing: 25.07.2002
(51) Int. Cl.: A61F 2/06

(54) **Devices for delivery of therapeutic agents with variable release profile**
Vorrichtungen zur Abgabe von therapeutischer Mittel mit variablem Freisetzungsprofil
Dispositif d'administration d'agents thérapeutiques à profil de libération variable

(30) Priority: 26.07.2001 US 308381 P; 01.11.2001 US 2595; 14.12.2001 US 17500; 10.01.2002 US 347473 P; 07.02.2002 US 355317 P; 06.04.2002 US 370703 P
(43) Date of publication of application: 12.05.2004
(73) Proprietor: Avantec Vascular Corporation, Sunnyvale, CA 94086 (US)
(72) Inventor: SIRHAN, Motasim, Sunnyvale, CA 94087 (US); YAN, John, Los Gatos, CA 95032 (US)
(74) Representative: Kazi, Ilya
(86) International application number: PCT/US2002/023830
(87) International publication number: WO 2003/009778

(56) References cited:
- EP-A- 0 950 386
- EP-A- 1 277 449
- WO-A-01/10421
- WO-A-98/36784
- WO-A-02/083039
- US-A- 5 024 671
- US-A- 5 545 208
- US-A- 5 843 172

## Description

### FIELD OF THE INVENTION

The present invention relates generally to medical devices and methods. More particularly, the present invention provides luminal prostheses, such as vascular stents and grafts for inhibiting restenosis.

### BACKGROUND OF THE INVENTION

A number of percutaneous intravascular procedures have been developed for treating stenotic atherosclerotic regions of a patient's vasculature to restore adequate blood flow. The most successful of these treatments is percutaneous transluminal angioplasty (PTA). In PTA, a catheter, having an expandable distal end, usually in the form of an inflatable balloon, is positioned in the blood vessel at the stenotic site. The expandable end is expanded to dilate the vessel to restore adequate blood flow beyond the diseased region. Other procedures for opening stenotic regions include directional arthrectomy, rotational arthrectomy, laser angioplasty, stenting, and the like. While these procedures have gained wide acceptance (either alone or in combination, particularly PTA in combination with stenting), they continue to suffer from significant disadvantages. A particularly common disadvantage with PTA and other known procedures for opening stenotic regions is the frequent occurrence of restenosis.

Restenosis refers to the re-narrowing of an artery after an initially successful angioplasty. Restenosis afflicts approximately up to 50% of all angioplasty patients and is the result of injury to the blood vessel wall during the lumen opening angioplasty procedure. In some patients, the injury initiates a repair response that is characterized by smooth muscle cell proliferation referred to as "hyperplasia° in the region traumatized by the angioplasty. This proliferation of smooth muscle cells re-narrows the lumen that was opened by the angioplasty within a few weeks to a few months, thereby necessitating a repeat PTA or other procedure to alleviate the restenosis.

A number of strategies have been proposed to treat hyperplasia and reduce restenosis. Previously proposed strategies include prolonged balloon inflation during angioplasty, treatment of the blood vessel with a heated balloon, treatment of the blood vessel with radiation following angioplasty, stenting of the region, and other procedures. While these proposals have enjoyed varying levels of success, no one of these procedures is proven to be entirely successful in substantially or completely avoiding all occurrences of restenosis and hyperplasia.

As an alternative or adjunctive to the above mentioned therapies, the administration of therapeutic agents following PTA for the inhibition of restenosis has also been proposed. Therapeutic treatments usually entail pushing or releasing a drug through a catheter or from a stent. While holding great promise, the delivery of therapeutic agents for the inhibition of restenosis has not been entirely successful.

Accordingly, it would be a significant advance to provide improved devices and methods for inhibiting restenosis and hyperplasia concurrently with or following angioplasty and/or other interventional treatments. This invention satisfies at least some of these and other needs.

EP-A-0950386 discloses an intravascular stent having micropores from which rapamycin may be delivered locally. EP-A-1277449 discloses a stent with recesses that are able to receive agents for the treatment of the site of implant of the stent. The recesses are made in such a way as to leave substantially unaltered the bending moments of respective elements of the stent. Also, WO-A-02/083039 discloses a stent of variable surface area having a patterned distribution of therapeutic agent that can be provided throughout the stent. Indentations may be provided on the surface of the stent with therapeutic agents disposed therein. A device as disclosed in the preamble of claim 1 is disclosed in WO-A-01/10 421.

### BRIEF SUMMARY OF THE INVENTION

As used herein, the following terms are defined as set forth below:

Associated with: Refers to any form of association such as directly or indirectly being coupled to, connected to, disposed on, disposed within, attached to, adhered to, bonded to, adjacent to, entrapped in, absorbed in, absorbed on, and like configurations

Inhibit: Includes any one of minimize, reduce, treat, contain, prevent, curb, eliminate, hold back, or restrain.

Intracorporeal body: Refers to body lumens or internal corporeal tissues and/or organs, within a corporeal body. The body lumen may be any blood vessel in the patient's vasculature, including veins, arteries, aorta, and particularly including coronary and peripheral arteries, as well as previously implanted grafts, shunts, fistulas, and the like. It will be appreciated that the present invention may also be applied to other body lumens, such as the biliary duct, which are subject to excessive neoplastic cell growth. Examples of internal corporeal tissues and organs applications include various organs, nerves, glands, ducts, and the like.

Intravascular intervention: Includes a variety of corrective procedures that may be performed to at least partially resolve a stenotic, restenotic, or thrombotic condition in a blood vessel, usually an artery, such as a coronary artery. Usually, the corrective procedure will comprise balloon angioplasty. The corrective procedure may also comprise directional atherectomy, rotational atherectomy, laser angioplasty, stenting, or the like, where the lumen of the treated blood vessel is enlarged to at least partially alleviate a stenotic condition which existed prior to the treatment.

Made available: To have provided the substance (e.g., therapeutic capable agent) at the time of release or administration, including having made the substance available at a corporeal location such as in intracorporeal location or target site, regardless of whether the substance is in fact delivered, used by, or incorporated into the intended site, such as the susceptible tissue site.

Radially expandable: Includes segments that can be converted from a small diameter configuration to a radially expanded, usually cylindrical, configuration which is achieved when an expandable structure is implanted at a desired target site.

Restenosis: Usually greater than 40% re-narrowing of the vessel diameter achieved by the vascular intervention.

Susceptible tissue site: A tissue site that is injured or may become injured as a result of an impairment (e.g., disease, medical condition), or may become injured during or following an interventional procedure such as an intravascular intervention. The susceptible tissue site may include tissues associated with intracorporeal lumens, organs, or localized tumors.

Therapeutic capable agent: Includes at least one compound, molecular species, and/or biologic agent that is either therapeutic as it is introduced to the subject under treatment, becomes therapeutic after entering the corporeal body of the subject, as for example by way of reaction with a native substance or condition as for example by way of reaction with a native or non-native substance or condition, or another introduced substance or condition. Examples of native conditions include pH (e.g., acidity), chemicals, temperature, salinity, and conductivity; with non-native conditions including those such as magnetic fields, and ultrasound. In the present application, the chemical name of any of the therapeutic capable agents or other compounds is used to refer to the compound itself and to pro-drugs (precursor substances that are converted into an active form of the compound in the body), and/or pharmaceutical derivatives, analogues, or metabolites thereof (bio-active compound to which the compound converts within the body directly or upon introduction of other agents or conditions (e.g., enzymatic, chemical, energy), or environment (e.g., pH)).

The present invention is directed to improved devices, more particularly intracorporeal devices, and methods for preparation or treatment of susceptible tissue sites, in an embodiment, the device includes a luminal prosthesis such as a vascular stent or graft, in one embodiment, the present devices and methods inhibit the formation or progression of restenosis and/or hyperplasia which may follow an intravascular intervention.

The invention provides a device as defined in Claim 1. Preferred features of the device are defined in the dependent claims.

Normally, the device, such as a coronary steal, is selected to have a length at least equal to a length of an injured site (eg., lesion) so as to extend the entire length of the lesion, preferably extending over the lesion. However, in some instances, stenosis is known to have been developed or increased at edges of the stent and/or beyond the stented or covered tissue area. This phenomenon is known as "edge effect" or candy wrapper effect. In patients experiencing the edge effect, although the stented portion may remain fice of significant restenosis, the site at or beyond the edges of the stent may develop significant or even severe stenosis, requiring subsequent treatment. The severity of the stenosis at the edge and/or beyond edge areas is usually greater at an area proximal to the stent as compared to an area distal to the stent. The occurrence of edge effect may be attributable to uncovered diseased segments subjected to balloon trauma which are not subsequently covered by the stent, migration of smooth cells from the lesioned area, injury during the interventional procedure (e.g., balloon injury during angioplasty with or without stenting), or the insufficient coverage of the original lesion. In the case of drug eluting stents, as described in more detail in U.S. Patent No. 6,602,281, such effect may further be attributable to drastic gradient change in a drug concentration between areas directly exposed to the drug (e.g., directly exposed to the stent including the drug) and areas that are not directly exposed to the drug.

In an embodiment, the devices and methods of the present invention particularly inhibit hyperplasia and/or restenosis at a stented area (i.e., in stent restenosis or ISR) as well as areas of the vessel at and/or beyond edges of the stent (i.e., peri-stent area). The peri-stent area may include either or both areas longitudinally proximal and distal to the stent. Usually such peri-stent area has a longitudinal dimension of about five (5) millimeters on either end of the stent. The inhibition at the proximal peri-stent area may be same, less, or greater than that at the distal peri-stent area. In one embodiment, the inhibition may occur while allowing for generation of a small amount of cellularization, endothelialization, or neointima, preferably, in a controlled manner.

In an embodiment, the device includes a structure and at least one source of at least one therapeutic capable agent associated with the structure. The source is configured to provide the therapeutic capable agent to the susceptible tissue site at a variable release profile along the stent. The release of the therapeutic capable agent upon introduction of the device within the patient's body may be immediate or after a delayed period.

In one embodiment, the device, such as a stent, has a longitudinal dimension, proximal and distal end portions, and an intermediate portion disposed between the proximal and distal end portions. The release profile varies across the length of the stent (i.e., structure longitudinal dimension), being greater or higher at the end portions as compared to the intermediate portion. One or more of the various configurations described below (e.g., chemical, structural, mechanical) may be used to configure the device to provide the desirable release profile. To allow for the difference in the susceptibility of the tissue at proximal and distal peri-stent areas, the release profile at the two end portions may be different. For example, the release profile may be greater at the proximal end portion than at the distal end portion.

The variable release profile may be achieved by way of employing different chemical, structural, or mechanical configurations. The chemical configuration may include any one or more of the following factors, but is not limited to: a particular therapeutic capable agent's diffusion properties as may be affected by properties such as molecular size, molecular weight, hydrophilicity, steric properties (e.g., size and spatial configuration), and nature of chemical substituents on the therapeutical capable agent. By way of example, a plurality of therapeutic capable agents may be employed such that the therapeutic capable agent with a higher diffusion rate is disposed at the end portions of the stent as compared to the therapeutic capable agent employed at the intermediate portion. Still further, the therapeutic capable agent at the intermediate portion may be less hydrophillic, have a larger molecular configuration, and/or have a higher molecular weight than the therapeutic capable agent at the end portions. The source at the intermediate portion may also include a higher mass of the therapeutic capable agent or different compositon than at the end portions.

Alternatively, for the same therapeutic capable agent active compound, various forms of the compound with different solubility rates may be used to impart variable release profile along the device. For example, the therapeutic capable agent at the end portions may comprise a more soluble salt form (e.g., mycophenolic acid and the sodium salt form of mycophenolic acid), an acidified form (e.g., benidipine and benidipine hydrochloride), or a more water soluble analog or derivative of the therapeutic capable agent (e.g., rapamycin and the more water soluble analog, such as, CCI-779 (available from Wyeth), methoxylpoly(ethyleneglycol) esters of rapamycin, aminoacyl prodrug of rapamycin, 42-oxorapamycin, 27-oximes of rapamycin, and other ester forms of rapamycin) than at the intermediate portion (e.g., non-salt form or non-acidified form of the therapeutic capable agent). As such, the therapeutic capable agent is more soluble in the patient's bodily fluids than the therapeutic capable agent at the intermediate portion.

By way of example, structural configurations effectuating different release profiles include: a location of the therapeutic capable agent on the stent, an initial amount of therapeutic capable agent loaded onto the stent, or utilizing a rate-controlling element. The therapeutic capable agent may be associated at least in part with either or both the structure and the rate-controlling element. The rate limiting element may be used either or both as a layer disposed adjacent, as for example over the source, thus providing a different release rate (e.g., mass/time) for the therapeutic capable agent, and as a matrix material used to form a matrix with the therapeutic capable agent. An amount of the rate-controlling element may be varied (e.g., thickness or concentration) over the length of the device, with the amount being greater at the intermediate portion (thus creating a lower release profile for the therapeutic capable agent). The end portions may have a lower amount of the rate-controlling element or none at all (e.g., the rate-controlling element is only present at the structure intermediate portion). The effect of the rate-controlling element on the release profile of the therapeutic capable agent may also be affected by other attributes of the rate-controlling element, such as its particular chemistry or morphology (e.g., porous versus non-porous).

In one embodiment the therapeutic capable agent at the end portions may be present without any rate-controlling element (or the therapeutic capable agent may be present as an outer most layer on the structure). The surface of the therapeutic capable agent may be smooth or textured. Such surface characteristic will also have an impact on the release profile of the therapeutic capable agent.

By way of example, mechanical configurations effectuating different release profiles include the design and material properties of the structure, such as a stent. The structure may be an expandable structure implantable within a corporeal body which includes the susceptible tissue site. The structure may have a substantially constant size or diameter, or alternatively depending on the application and use, may be a contractable structure. The structure may include at least one surface, usually, a tissue facing (i.e., abluminal surface) surface, normally both a tissue facing surface and a lumen facing surface. An exemplary stent for use in the present invention is described in U.S. Patent No. 6,602,282 assigned to the assignee of the present application.

The stent generally includes a cylindrical frame having proximal and distal ends and tissue and luminal facing surfaces. The stent usually further comprises a plurality of radially expansible unit segments including rings. The rings preferably have a serpentine shape. In an embodiment, the unit segments preferably include segments having different mechanical profiles, as for example may be exhibited as a result of expansion. In an embodiment, some of the rings may be joined with at least one axially adjacent ring through expansion links. The links preferably have a sigmoidal shape, more preferably, an S shape having a relatively smooth profile along its length to minimize or reduce kinking upon expansion. Similarly, the links may comprise segments having different mechanical profiles along their length.

The structure may include portions having different mechanical stress or strain profiles upon expansion, contraction, or areas which are substantially in a direct line of fluid (e.g., blood or other bodily fluids) flow through the body. The different portions of the structure may have relatively lower and relatively higher mechanical stress or strain profiles with respect to one another and exhibit different stress characteristics during expansion of the device when implanted within the intracorporeal body. As used herein, the term "having different mechanical profile" refers to this characteristic of the structure or prosthesis. For example, the unit segments and/or links may have relatively lower mechanical profile portions along their lengths with relatively higher mechanical profile portions at bends, points, intersections, joints, or areas exposed to flow turbulence.

The variable release profile may be achieved by disposing the source at the structure segments with relatively higher mechanical profiles at the end portions and the source at the structure segments with relatively lower mechanical profiles at the intermediate portion. This will bring about a higher release profile at the end portions than at the intermediate portion.

The expandable structure may be formed of any suitable material such as metals, polymers, or a combination thereof. In an embodiment, the structure may be formed from malleable metals or alloys, such as 300 series stainless steel; resilient metals, such as superelastic and shape memory alloys (e.g., nitinol alloys, spring stainless steels, and the like); non-metallic materials, such as ceramics or polymeric materials; or a combination thereof.

In one embodiment, the expandable structure may be formed of an at least partially biodegradable material selected from the group consisting of polymeric material, metallic materials, ceramic materials, or combinations thereof. The at least partially biodegradable material preferably degrades over time. Examples of polymeric material include poly-L-lactic acid, having a delayed degradation to allow for the recovery of the vessel before the structure is degraded. Example of metallic material include metals or alloys degradable in the corporeal body, such as stainless steel. Other suitable material for use as the structure include carbon or carbon fiber, cellulose acetate, cellulose nitrate, silicone, polyethylene terphthalate, polyurethane, polyamide, polyester, polyorthoester, polyanhydride, polyether sulfone, polycarbonate, polytetrafluoroethylene, another biocompatible polymeric materials, polyanhydride, polycaprolactone, polyhydroxybutyrate valerate, another biodegradable polymer, protein, an extracellular matrix component, collagen, fibrin, another biologic agent, or a suitable mixture or copolymer of any of the materials listed above, degradable, non-degradable, metallic, or otherwise.

The rate-controlling element may be formed of a non-degradable, partially degradable, substantially degradable material, or a combination thereof. The material may be synthetic or natural; non-polymeric, polymeric, ceramic, or metallic; bio-active or non bio-active compounds; or a combination thereof. The rate-controlling element may have a porous, microporous, nanoporous, or non-porous morphology or any combinations thereof. Preferably, when the device comprises a porous rate-controlling element, at least one layer of a non-porous rate-controlling element is disposed between the source and the porous rate-controlling element.

In a preferred embodiment, the rate-controlling element is formed from a non-porous material, usually a non-porous conformal material. Example of suitable non-porous material include, but is not limited to: plasma deposited polymers; sputtered, evaporated, electroplated metals and/or alloys; glow discharge coating; polyethylene; polyurethanes; silicone rubber; cellulose; and parylene including parylene C, N, D, and F, or combinations thereof, usually parylene C, preferably non-porous parylene C.

The therapeutic capable agent may be selected from a group consisting of immunosuppressants, anti-inflammatories, anti-proliferatives, anti-migratory agents, anti-fibrotic agents, proapoptotics, vasodilators, calcium channel blockers, anti-neoplastics, anticancer agents, antibodies, anti-thrombotic agents, anti-platelet agents, IIb/IIIa agents, antiviral agents, mTOR (mammalian target of rapamycin) inhibitors, non-immunosuppressant agents, and a combination thereof. Specific examples of therapeutic capable agent include: mycophenolic acid, mycophenolic acid derivatives (e.g., 2-methoxymethyl derivative and 2-methyl derivative), VX-148, VX-944, mycophenolate mofetil, mizoribine, methylprednisolone, dexamethasone, CERTICAN^{™} (e.g., everolimus, RAD), rapamycin, ABT-773 (Abbot Labs), ABT-797 (Abbot Labs), TRIPTOLIDE^{™}, METHOTREXATE^{™}, phenylalkylamines (e.g., verapamil), benzothiazepines (e.g., diltiazem), 1,4-dihydropyridines (e.g., benidipine, nifedipine, nicarrdipine, isradipine, felodipine, amlodipine, nilvadipine, nisoldipine, manidipine, nitrendipine, bamidipine (HYPOCA^{™})), ASCOMYCIN^{™}, WORTMANNIN^{™}, LY294002, CAMPTOTHECIN^{™}, flavopiridol, isoquinoline, HA-1077 (1-(5-isoquinolinesulfonyl)-homopiperazine hydrochloride), TAS-301 (3-bis(4-methoxyphenyl)methylene-2-indolinone), TOPOTECAN^{™}, hydroxyurea, TACROLIMUS^{™} (FK 506), cyclophosphamide, cyclosporine, daclizumab, azathioprine, prednisone, diferuloymethane, diferuloylmethane, diferulylmethane, GEMCITABINE^{™}, cilostazol (PLETAL^{™}, tranilast, enalapril, quercetin, suramin, estradiol, cycloheximide, tiazofurin, zafurin, AP23573 (an analogue ofrapamycin available from Ariad Pharmaceuticals), CCI-779 (an analogue of rapamycin available from Wyeth), sodium mycophenolic acid, benidipine hydrochloride, sirolimus, rapamune, rapamycin derivatives, non-immunosuppressive analogues of rapamycin (e.g., rapalog, AP21967, derivatives of rapalog), metabolites, derivatives, and/or combinations thereof.

The devices of the present invention may be provided together with instructions for use (IFU), separately or as part of a kit. The kit may include a pouch or any other suitable package, such as a tray, box, tube, or the like, to contain the device and the IFU, where the IFU may be printed on a separate sheet or other media of communication and/or on the packaging itself. In an embodiment, the kit may also include a mounting hook, such as a crimping device and/or an expansible inflation member, which may be permanently or releaseably coupled to the device of the present invention.

In operation, methods of delivering the therapeutic capable agent to the susceptible tissue site comprise positioning the source of the therapeutic capable agent within the intracorporeal site, such as the vascular lumen. The therapeutic capable agent is then released and/or made available to the susceptible tissue site.

Methods of treatment generally include positioning the source including the at least one therapeutic capable agent and/or an optional another compound within the intracorporeal body, concurrently with, or subsequent to, an interventional treatment. More specifically, the therapeutic capable agent may be delivered to a targeted corporeal site (e.g., targeted intracorporeal site) which includes the susceptible tissue site or a targeted site providing the therapeutic capable agent to the susceptible tissue site, concurrently with or subsequent to the interventional treatment. By way of example, following the dilation of the stenotic region with a dilatation balloon, a device (such as a stent) according to the present invention, is delivered and implanted in the vessel. The therapeutic capable agent may be made available to the susceptible tissue site at amounts which may be sustainable, intermittent, or continuous; at one or more phases; and/or rates of delivery.

### BRIEF DESCRIPTION OF THE DRAWINGS

FIG. 1 is a cross sectional view of a device implanted in a body lumen.

FIG. 2 is a schematic representation of an exemplary stent.

FIGS. 3 through and 3G are cross sectional views of different embodiments of the stent of FIG. 2.

FIGS. 4, 4A, and 4B are schematic representations of an expanded view of a portion of the stent of FIG. 2 showing areas having different mechanical profiles.

### DETAILED DESCRIPTION OF THE INVENTION

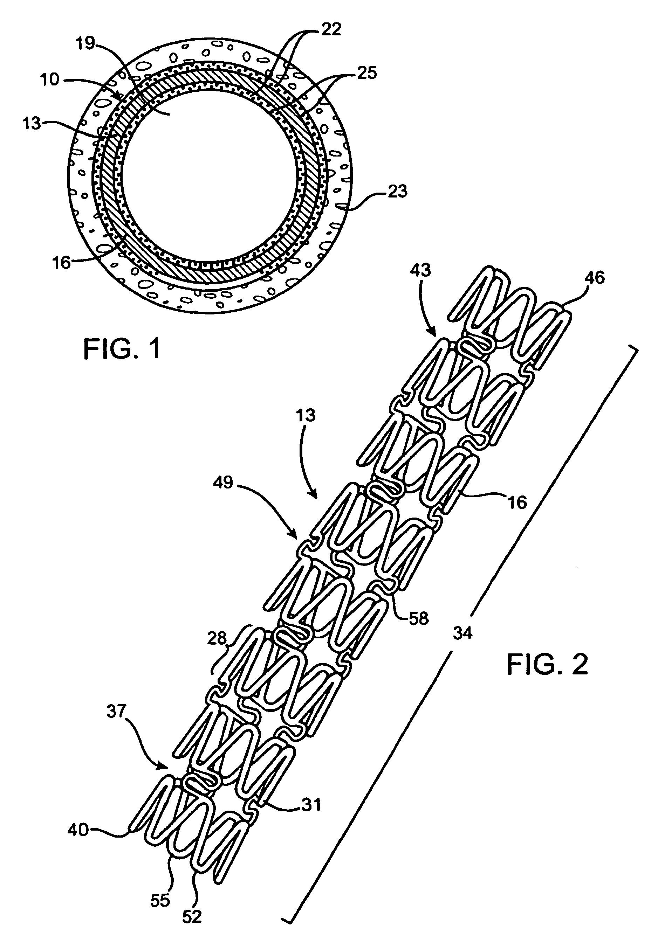

FIGS. 1 and 2 illustrate a device 10, such as a prosthesis 13, generally including an expandable structure 16 implantable in an intracorporeal body, such as body lumen 19 including a susceptible tissue site 23, and a source 22 adjacent the expandable structure 16 and including a therapeutic capable agent 25. The source may be disposed on one or both surfaces of the expandable structure.

The prosthesis 13 generally includes a plurality of radially expansible unit segments including rings 28, a cylindrical frame 31 having a longitudinal dimension 34, a proximal end portion 37 having a proximal end 40, a distal portion 43 having a distal end 46, an intermediate portion 49 disposed between the end portion ends, and tissue and luminal facing surfaces, 52 and 55.

. When the prosthesis is a stent, the expandable structure 16 will usually comprise at least two radially expandable, usually cylindrical, ring segments 28 as shown in FIG. 2. Typically, the expandable structure 16 will have at least four, and often five, six, seven, eight, nine, ten, or more ring segments. At least some of the ring segments will be adjacent to each other, but other ring segments may be separated by other non-ring structures. The description of exemplary stent structures is not intended to be exhaustive and it should be appreciated that other variations of stent designs may be used in the present invention.

The exemplary stent 13 (embodying features of a stent described in more detail in U.S. Patent No. 6,602,281) comprises from 4 to 50 ring segments 28 (with eight being illustrated). At least some of the rings 28, as shown, are joined with at least one axially adjacent ring through expansion links 58, preferably having a sigmoidal shape, more preferably an S shape having a relatively smooth profile along its length to minimize or reduce kinking upon expansion. Preferably, the rings 28, as shown, have a serpentine shape.

As shown, each ring segment 28 is joined to the adjacent -ring segment by at least one of sigmoidal links 58 (with three being illustrated). Each ring segment 28 includes a plurality of strut/hinge units, e.g., six, strut/hinge units, and three out of each six hinge/strut structures on each ring segment 28 will be joined by the sigmoidal links 58 to the adjacent ring segment. As shown in FIG. 2, the stent 16 is in a collapsed or non-expanded configuration.

The radially expandable structure includes segments that can be converted from a small diameter configuration to a radially expanded, usually cylindrical, configuration which is achieved when the expandable structure 16 is implanted at a desired target site. The expandable structure 16 may be minimally resilient, e.g., malleable, thus requiring the application of an internal force to expand and set it at the target site. Typically, the expansive force can be provided by a balloon, such as the balloon of an angioplasty catheter for vascular procedures. The expandable structure 16 preferably provides sigmoidal links between successive unit segments which are particularly useful to enhance flexibility and crimpability of the stent.

Alternatively, the expandable structure 16 can be self-expanding. Self-expanding structures are provided by utilizing a resilient material, such as a tempered stainless steel, or a superelastic alloy such as a nitinol alloy, and forming the body segment so that it possesses a desired radially-expanded diameter when it is unconstrained, i.e, released from the radially constraining forces of a sheath. In order to remain anchored in the body lumen, the expandable structure 16 will remain partially constrained by the lumen. The self-expanding expandable structure 16 can be tracked and delivered in its radially constrained configuration, e.g., by placing the expandable structure 16 within a delivery sheath or tube and removing the sheath at the target site.

The stent 13 across its length 34 has a variable release profile for the therapeutic capable agent, with the release profile at the end portions 37 and 43 being preferably greater than the release profile at the intermediate portion 49. The variable release profile may be achieved by way of employing any one or more of chemical, structural, or mechanical configurations described above.

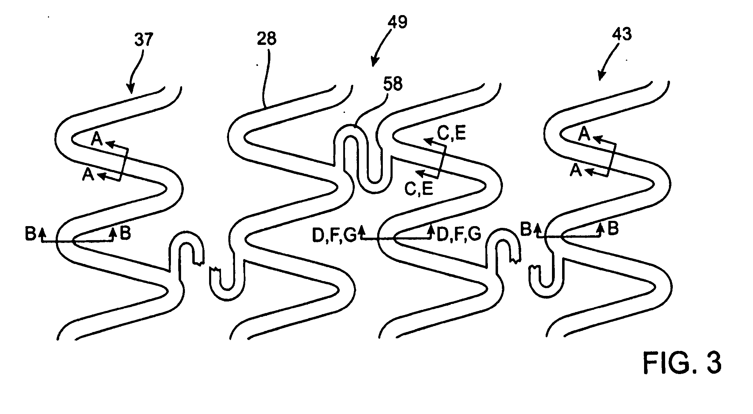

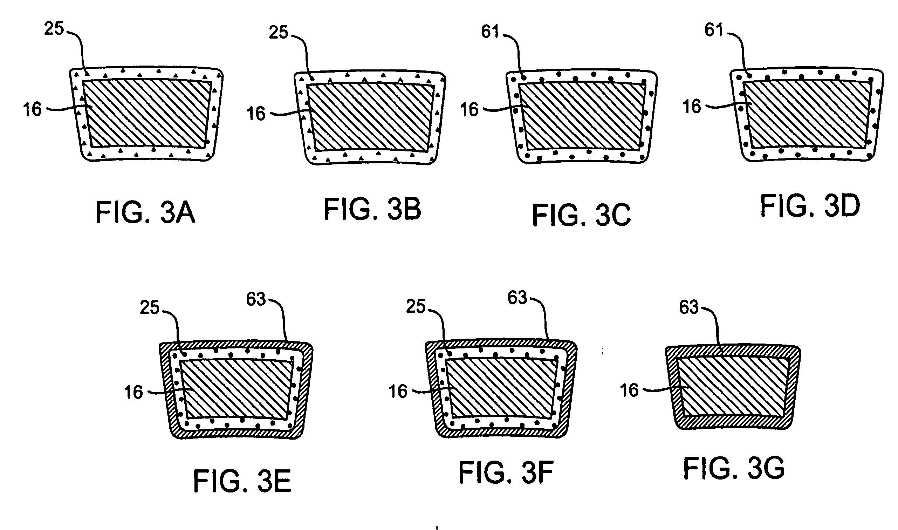

Now referring to FIGS. 3, 3A, 3B, 3C, and 3D, the source at the end portions 37 and 43 may include at least one therapeutic capable agent 25 and at the intermediate portion 43 may include at least one other or different therapeutic capable agent 61, with the two therapeutic capable agents 25, 61 having different chemical properties to effectuate different release profiles along the length 34 of the stent 16. Preferably, the end portions will have a higher release profile.

By way of example, therapeutic capable agents such as mycophenolic acid, methylprednisolone, TACROLIMUS^{™}, benidipine, rapamycin, sirolimus, rapamune, have different levels of hydrophilicity, with the list being in a decreasing order of hyrophilicity. The release profile may also be affected by the size and molecular weight of the therapeutic capable agent. For example, the larger size and higher molecular weight of rapamycin may slow down its diffusion through a rate-controlling element and/or tissue as compared to mycophenolic acid. As such, this results in a lower release profile for the rapamycin.

The size and nature of substituents may also affect the release profile of the therapeutic capable agent. Therapeutic capable agents with phenyl or high molecular weight aliphatic chains (e.g., benidipine with phenyl substituents) tend to diffuse slower than therapeutic capable agents with methyl and hydroxyl substitutes (e.g., mycophenolic acid with methyl and hydroxyl substituents), thus exhibiting a lower release profile.

FIGS. 3E and 3F illustrate features of an embodiment of the stent of the present invention, further including a rate-controlling element 63 disposed adjacent and over the therapeutic capable agent 25 at the intermediate portion 49, with the end portions 37 and 43 being free from the rate-controlling element. The absence of the rate-controlling element at the end portions provides for a higher release profile at the end portions as compared to the intermediate portion. The rate-controlling element may be disposed as a layer adjacent and over the therapeutic capable agent, as shown in FIGS. 3E and 3F, or additionally and/or alternatively be used as a matrix material mixed with the therapeutic capable agent and forming a matrix therewith. FIG. 3H illustrates the rate-controlling element disposed as a layer 63 adjacent and over the structure 16 at the intermediate portion 49.

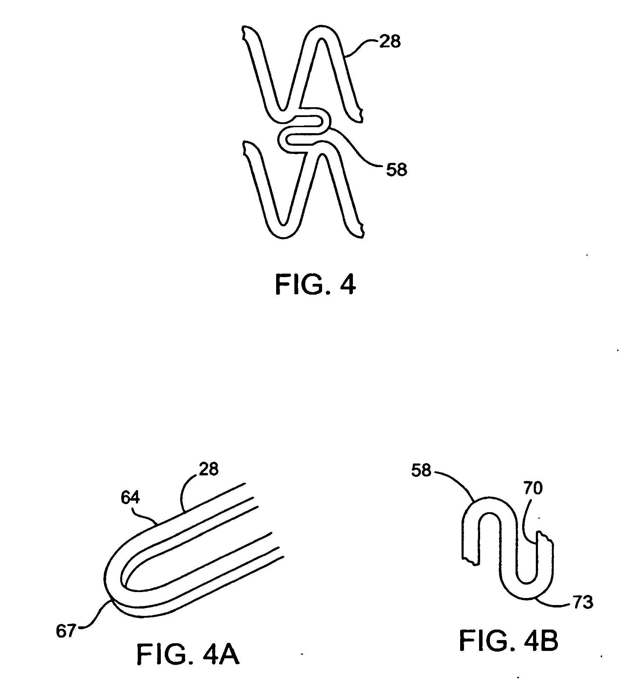

Now referring back to FIG. 2 and FIGS. 4, 4A, and 4B, the unit segments 28 preferably include segments having different mechanical profiles, as for example may be exhibited as a result of expansion. For example, the segments 28 may include relatively lower mechanical profile portions 64 along their lengths with relatively higher mechanical profile portions 67 at bends, points, intersections, joints, or areas exposed to flow turbulence (FIG. 4A). The areas exhibiting relatively lower mechanical profiles, upon expansion of the expandable structure, typically do not undergo substantial bending, flexing, stretching, or compression, usually being less than about 5%. Similarly, the links 58 may comprise segments having different mechanical profile profiles along their length (FIG. 4B). For example, the links 58 may include relatively lower mechanical profile portions 70 along their lengths with relatively higher mechanical profile portions 73 at bends, points, intersections, joints, or areas exposed to flow turbulence (i.e., areas which are substantially in the direct line of fluid (e.g., blood or other bodily fluids) flow through the body).

The variable release profile may be achieved by disposing the source at the structure segments with relatively higher mechanical profiles at the end portions, as shown in Fig. 3B, and the source at the structure segments with relatively lower mechanical profiles at the intermediate portion, as shown in FIG. 3E. This will bring about a higher release profile at the end portions than at the intermediate portion.

One or more of the various configurations described above (e.g., chemical, structural, mechanical) may be used to configure the device to provide the desired release profile. By way of example, the stent may include a therapeutic capable agent with a higher diffusion rate at the end portions than at the intermediate portion.

The dimensions of the expandable structure will depend on its intended use. Typically, the expandable structure will have a length in a range from about 5 mm to about 100 mm, usually being from about 8 mm to about 50 mm, for vascular applications. The diameter of a cylindrically shaped expandable structure for vascular applications, in a non-expanded configuration, usually ranges from about 0.5 mm to about 10 mm, more usually from about 0.8 mm to about 8 mm; with the diameter in an expanded configuration ranging from about 1.0 mm to about 100 mm, preferably from about 2.0 mm to about 30 mm. The expandable structure usually will have a thickness in a range from about 0.025 mm to 2.0 mm, preferably from about 0.05 mm to about 0.5 mm. The length of the end portions may be anywhere from about 0 to about 15% of the total length of the structure, preferably from about 0.1% to about 10% of the total length of the structure, most preferably from about 1% to about 5% of the total length of the structure.

The expandable structure may include the therapeutic capable agent by coating, spraying, dipping, deposition (vapor or plasma), or painting the therapeutic capable agent onto the prosthesis. Usually, the therapeutic capable agent is dissolved in a solvent prior to its application. Suitable solvents include aqueous solvents (e.g., water with pH buffers, pH adjusters, organic salts, and inorganic salts), alcohols (e.g., methanol, ethanol, propanol, isopropanol, hexanol, and glycols), nitriles (e.g., acetonitrile, benzonitrile, and butyronitrile), amides (e.g., formamide and N-dimethylformamide), ketones, esters, ethers, DMSO, gases (e.g., CO₂), and the like. The therapeutic capable agent structure is then allowed to dry. Alternatively, the therapeutic capable agent may first be prepared into a matrix by mixing or dissolving the therapeutic capable agent and matrix material, alone or in combination with a solvent, prior to its incorporation to the structure. When desired, a masking technique may be utilized to provide for selective coating of the therapeutic capable agent or other material on the structure.

## Claims

1. A device for intracorporeal use within a patient's body, the device comprising:
a structure (13, 16) having a longitudinal dimension (34) defined by proximal and distal end portions (37, 43) and an intermediate portion (49) disposed therebetween;
at least one source (22) of at least one therapeutic capable agent (25) associated with the structure (13, 16); and
a rate-controlling element (63) disposed adjacent at least a portion of the source (22), **characterized in that** it is configured to the therapeutic capable agent (25) at a longitudinally variable release profile along the structures longitudinal dimension (34), wherein the therapeutic capable agent (25) release profile is greater at the end portions (37, 43) than the therapeutic capable agent (25) release profile at the intermediate portion (49).

2. A device as in Claim 1, wherein the release profile is greater at the proximal end portion (37) than at the distal end portion (43).

3. A device as in Claim 1 or Claim 2, wherein the source (22) at the intermediate portion (49) has a different composition than the source (22) at the end portions (37, 43).

4. A device as in any one of Claims 1-3, wherein the therapeutic capable agent (25) at one or both end portions (37. 43) has a higher diffusion rate into the intracorporeal body than the therapeutic capable agent (25) at the intermediate portion (49).

5. A device as in any one of Claims 1-4, wherein the therapeutic capable agent (25) at the intermediate portion (49) is less hydrophilic, has a larger molecular configuration, has a higher molecular weight, or is otherwise different than the therapeutic capable agent (25) at the end portions (37, 43).

6. A device as in any one of Claims 1-5, wherein the therapeutic capable agent (25) at one or both end portions (37, 43) is more soluble in the patient's bodily fluids than the therapeutic capable agent (25) at the intermediate portion (49).

7. A device as in any one of Claims 1-6, wherein the therapeutic capable agent (25) at one or both end portions (37, 43) is a more soluble analog form or derivative of the therapeutic capable agent (25) as compared to the intermediate portion (49).

8. A device as in any one of Claims 1-7, wherein the therapeutic capable agent (25) at one or both end portions (37, 43) is a salt, or acidified form of the therapeutic capable agent (25) and the therapeutic capable agent (25) at the intermediate portion (49) is a non-salt, or non-acidified form of the therapeutic capable agent (25).

9. A device as in any of Claims 1-8, wherein the therapeutic capable agent (25) comprises mycophenolic acid, a sodium form of mycophenolic acid, benidipine, benidipine hydrochloride, rapamycin, CCI-779, metabolites, derivatives, or combinations thereof.

10. A device as in any one of Claims 1-9, wherein the at least one source (22) is configured to provide the therapeutic capable agent (25) at an amount effective to inhibit neointimal hyperplasia at an area longitudinally adjacent the structure (13, 16).

11. A device as in Claim 11, wherein the adjacent area includes at least one of proximally or distally adjacent areas to the structure (13, 16), or is within and including 5 millimeters from one or each end (40, 46) of the structure (13, 16).

12. A device as in any one of Claims 1-11, wherein the rate-controlling element (63) has a variable thickness across the longitudinal dimension (34).

13. A device as in any one of Claims 1-11, wherein the rate-controlling element (63) is disposed only adjacent the intermediate portion (49).

14. A device as in any one of Claims 1-11, wherein the rate-controlling element (63) at the intermediate portion (49) is greater than at the structure end portions (37, 43).

## Patentansprüche

1. Vorrichtung zur intrakorporalen Verwendung im Körper eines Patienten, die aufweist:
eine Struktur (13,16) mit einer Längsdimension (34), die durch proximale und distale Endabschnitte (37, 43) und einen dazwischen angeordneten Zwischenabschnitt (49) definiert ist;
wenigstens eine Quelle (22) wenigstens eines therapiegeeigneten Mittels (25), die mit der Struktur (13, 16) verbunden ist; und
ein Geschwindigkeitssteuerungs-Element (63), das neben wenigstens einem Abschnitt der Quelle (22) angeordnet ist,
**dadurch gekennzeichnet, dass**
sie so konfiguriert ist, dass sie das therapiegeeignete Mittel (25) an einem der Länge nach variablen Freisetzungsprofil entlang der Längsdimension (34) der Struktur freisetzen kann, wobei das Freisetzungsprofil (25) für das therapiegeeignete Mittel (25) an den Endabschnitten (37, 43) größer ist, als das Freisetzungsprofil des therapiegeeigneten Mittels (25) an dem Zwischenabschnitt (49).

2. Vorrichtung nach Anspruch 1, wobei das Freisetzungsprofil am proximalen Endabschnitt (37) größer ist, als am distalen Endabschnitt (43).

3. Vorrichtung nach Anspruch 1 oder Anspruch 2, wobei die Quelle (22) an dem Zwischenabschnitt (49) eine andere Zusammensetzung aufweist, als die Quelle (22) an den Endabschnitten (37, 43).

4. Vorrichtung nach einem der Ansprüche 1 bis 3, wobei das therapiegeeignete Mittel (25) an einem oder beiden Endabschnitten (37, 43) eine höhere Diffusionsgeschwindigkeit in den intrakorporalen Körper aufweist, als das therapiegeeignete Mittel (25) an dem Zwischenabschnitt (49).

5. Vorrichtung nach einem der Ansprüche 1 bis 4, wobei das therapiegeeignete Mittel (25) an dem Zwischenabschnitt (49) weniger hydrophil ist, eine größere Molekularkonfiguration aufweist, ein höheres Molekulargewicht auf weist, oder anderweitig anders ist, als das therapiegeeignete Mittel (25) an den Endabschnitten (37, 43).

6. Vorrichtung nach einem der Ansprüche 1 bis 5, wobei das therapiegeeignete Mittel (25) an einem oder beiden Endabschnitten (3 7, 43) in den Körperflüssigkeiten des Patienten besser löslich ist, als das therapiegeeignete Mittel (25) an dem Zwischenabschnitt (49).

7. Vorrichtung nach einem der Ansprüche 1 bis 6, wobei das therapiegeeignete Mittel (25) an einem oder beiden Endabschnitten (3 7, 43) eine besser lösliche analoge Form oder Derivativ des therapiegeeigneten Mittels (25) im Vergleich zu dem Zwischenabschnitt (49) ist.

8. Vorrichtung nach einem der Ansprüche 1 bis 7, wobei das therapiegeeignete Mittel (25) an einem oder beiden Endabschnitten (3 7, 43) ein Salz oder eine gesäuerte Form des therapiegeeigneten Mittels (25) ist, und wobei das therapiegeeignete Mittel (25) an dem Zwischenabschnitt (49) ein Nicht-Salz oder eine nicht-gesäuerte Form des therapiegeeigneten Mittels (25) ist.

9. Vorrichtung nach einem der Ansprüche 1 bis 8, wobei das therapiegeeignete Mittel (25) Mycophenolsäure, eine Natriumform von Mycophenolsäure, Benidipin, Benidipin-Hydrochlorid, Rapamycin, CC1-779, Metaboliten, Derivative oder Kombinationen hiervon umfasst.

10. Vorrichtung nach einem der Ansprüche 1 bis 9, wobei die wenigstens eine Quelle (22) so konfiguriert ist, dass sie das therapiegeeignete Mittel (25) in einer Menge, die wirksam ist, um neointimale Hyperplasie in einem Bereich längs benachbart der Struktur (13,16) zu verhindern, bereitstellen kann.

11. Vorrichtung nach Anspruch 10, wobei der benachbarte Bereich wenigstens einen der proximal oder distal benachbarten Bereiche der Struktur (13,16) umfasst oder innerhalb einschließlich 5 Millimetern von einem oder jedem Ende (40, 46) der Struktur (13,16) liegt.

12. Vorrichtung nach einem der Ansprüche 1 bis 11, wobei das Geschwindigkeitssteuerungs-Element (63) über die Längsdimension (34) eine variable Stärke aufweist.

13. Vorrichtung nach einem der Ansprüche 1 bis 11, wobei das Geschwindigkeitssteuerungs-Element (63) nur nahe des Zwischenabschnitts (49) angeordnet ist.

14. Vorrichtung nach einem der Ansprüche 1 bis 11, wobei das Geschwindigkeitssteuerungs-Element (63) an dem Zwischenabschnitt (49) größer ist, als an den Endabschnitten (37, 43) der Struktur.

## Revendications

1. Dispositif pour une utilisation intracaverneuse dans le corps d'un patient, le dispositif comprenant :
une structure (13, 16) ayant une dimension longitudinale (34) définie par des parties d'extrémité proximale et distale (37, 43) et une partie intermédiaire (49) disposée entre elles :
au moins une source (22) d'au moins un agent thérapeutique approprié (25) associé à la structure (13, 16) ; et
un élément de régulation de vitesse (63) disposé de manière adjacente à au moins une partie de la source (22), **caractérisé en ce qu'**il est configuré pour libérer l'agent thérapeutique approprié (25) à un profil de libération variable longitudinalement le long de la dimension longitudinale de la structure (34), dans lequel le profil de libération de l'agent thérapeutique approprié (25) est plus grand au niveau des parties d'extrémité (37, 43) que le profil de libération de l'agent thérapeutique approprié (25) au niveau de la partie intermédiaire (49).

2. Dispositif selon la revendication 1, dans lequel le profil de libération est plus grand au niveau de la partie d'extrémité proximale (37) qu'au niveau de la partie d'extrémité distale (43).

3. Dispositif selon la revendication 1 ou 2, dans lequel la source (22) au niveau de la partie intermédiaire (49) présente une composition différente de la source (22) au niveau des parties d'extrémité (37, 43).

4. Dispositif selon l'une quelconque des revendications 1 à 3, dans lequel l'agent thérapeutique approprié (25) au niveau d'une ou des deux parties d'extrémité (37, 43) présente une vitesse de diffusion plus élevée dans le corps intracaverneux que l'agent thérapeutique approprié (25) au niveau de la partie intermédiaire (49).

5. Dispositif selon l'une quelconque des revendications 1 à 4, dans lequel l'agent thérapeutique approprié (25) au niveau de la partie intermédiaire (49) est moins hydrophile, présente une configuration moléculaire plus grande, présente un poids moléculaire plus élevé ou est autrement différent de l'agent thérapeutique approprié (25) au niveau des parties d'extrémité (37, 43).

6. Dispositif selon l'une quelconque des revendications 1 à 5, dans lequel l'agent thérapeutique approprié (25) au niveau d'une ou des deux parties d'extrémité (37, 43) est plus soluble dans les fluides corporels du patient que l'agent thérapeutique approprié (25) au niveau de la partie intermédiaire (49).

7. Dispositif selon l'une quelconque des revendications 1 à 6, dans lequel l'agent thérapeutique approprié (25) au niveau d'une ou des deux parties d'extrémité (37, 43) est une forme analogue plus soluble ou un dérivé de l'agent thérapeutique approprié (25) comparativement à la partie intermédiaire (49).

8. Dispositif selon l'une quelconque des revendications 1 à 7, dans lequel l'agent thérapeutique approprié (25) au niveau d'une ou des deux parties d'extrémité (37, 43) est une forme saline ou acidifiée de l'agent thérapeutique approprié (25) et l'agent thérapeutique approprié (25) au niveau de la partie intermédiaire (49) est une forme non saline, non acidifiée de l'agent thérapeutique approprié (25).

9. Dispositif selon l'une quelconque des revendications 1 à 8, dans lequel l'agent thérapeutique approprié (25) comprend de l'acide mycophénolique, une forme saline d'acide mycophénolique, du bénidipine, de l'hydrochlorure de bénidipine, de la rapamycine, du CCI-779, des métabolites, des dérivés ou des combinaisons de ceux-ci.

10. Dispositif selon l'une quelconque des revendications 1 à 9, dans lequel au moins une source (22) est configurée pour fournir l'agent thérapeutique approprié (25) en une quantité efficace pour empêcher l'hyperplasie néointimale au niveau d'une zone adjacente longitudinalement à la structure (13, 16).

11. Dispositif selon la revendication 11, dans lequel la zone adjacente comprend au moins une des zones adjacentes de manière proximale ou distale à la structure (13, 16) ou est dans 5 millimètres inclus de l'une ou de chaque extrémité (40, 46) de la structure (13, 16).

12. Dispositif selon l'une quelconque des revendications 1 à 11, dans lequel l'élément de régulation de vitesse (63) présente une épaisseur variable à travers la dimension longitudinale (34).

13. Dispositif selon l'une quelconque des revendications 1 à 11, dans lequel l'élément de régulation de vitesse (63) est disposé adjacent uniquement à la partie intermédiaire (49).

14. Dispositif selon l'une quelconque des revendications 1 à 11, dans lequel l'élément de régulation de vitesse (63) au niveau de la partie intermédiaire (49) est plus grand qu'au niveau des parties d'extrémité de structure (37, 43).
